# EUROPEAN PATENT APPLICATION

(11) **EP 3 175 875 A1**
(43) Date of publication of application: **07.06.2017**
(21) Application number: 15197618.0
(22) Date of filing: 02.12.2015
(51) Int. Cl.: A61M 5/24

(54) **METHOD FOR ASSEMBLING A DRUG DELIVERY DEVICE AND DRUG DELIVERY DEVICE FORMED BY THE METHOD**

(71) Applicant: NOVO NORDISK A/S, 2880 Bagsværd (DK)
(72) Inventor: Bengtsson, Henrik, 2630 Taastrup (DK); Nørtoft Sørensen, Dan, 3450 Allerød (DK)

(57) **Abstract**

The present invention relates to a method of assembling a drug delivery device (100, 200) comprising an expelling mechanism for providing a force on the piston (114, 214) of a held cartridge (13, 113, 213). The method includes the steps of a) providing a cartridge (13, 113, 213) comprising a cartridge body having a distal outlet being closed by a penetrable septum (17) and having a proximal open end closed by a slidable piston (114, 214), b) providing a piston rod (120, 220) for transferring a force to the piston, c) arranging the cartridge and the piston rod along an axis, d) providing a monitoring arrangement (A, B) configured for detecting a change in geometry of the septum (17), and e) providing relative axial movement between the piston rod (120, 220) and the cartridge body for causing the piston rod to move the piston (114, 214) towards the septum (17) with the effect of deflecting the septum until the septum assumes a predetermined geometry change. The invention further relates to a drug delivery device assembled according to the method.

## Description

The present invention relates to an assembly of components for a drug delivery device that allows a user to select single or multiple doses of an injectable liquid drug and to dispense the set dose of the product and to apply said product to a patient, preferably by injection. In particular, the present invention relates to a method of assembling such drug delivery devices.

### BACKGROUND

In the disclosure of the present invention reference is mostly made to drug delivery devices used e.g. in the treatment of diabetes by delivery of insulin, however, this is only an exemplary use of the present invention.

Drug delivery devices allowing for multiple dosing of a required dosage of a liquid medicinal product, such as liquid drugs, and further providing administration of the liquid to a patient, are as such well-known in the art. Generally, such devices have substantially the same purpose as that of an ordinary syringe.

Drug delivery devices of this kind have to meet a number of user specific requirements. For instance in case of those with diabetes, many users will be physically infirm and may also have impaired vision. Therefore, these devices need to be robust in construction, yet easy to use, both in terms of the manipulation of the parts and understanding by a user of its operation. Further, the dose setting must be easy and unambiguous and where the device is to be disposable rather than reusable, the device should be inexpensive to manufacture and easy to dispose. In order to meet these requirements, the number of parts and steps required to assemble the device and an overall number of material types the device is made from have to be kept to a minimum.

Typically, the liquid drug to be administered is provided in a cartridge that has a moveable piston or bung mechanically interacting with a piston rod driven by an expelling mechanism of the drug delivery device. By applying thrust to the piston in distal direction, a predefined amount of the liquid drug is expelled from an outlet of the cartridge. Due to inevitable manufacturing tolerances there may for instance persist axial clearance between a cartridge's piston and the piston rod.

Typically, prior to a primary use of the device, an end-user has to conduct a so-called priming of the expelling mechanism in order to ensure that already with an initial dose setting and a first subsequent dose dispensing step, an accurate amount of the liquid drug is dispensed in a predefined way. An initial dose setting and expelling of a minor dose may in certain situations also be required for removing any air present in the cartridge and/or a connected needle and for performing a flow check.

Document WO 99/38554 A1 discloses several embodiments of injection devices suitable for being provided as disposable devices wherein a liquid drug cartridge is inserted into the device during assembly in a production line.

In the production line, during final assembly operations of the device, at least a part of the priming is typically carried out using the dose setting and expelling mechanism so that users will experience virtually consistent requirement for a priming operation across individual pen samples. However, as disclosed in WO 2009/095332 A1, devices may include other adjustable means that are subject to adjustment during assembly so that the distance between the distal end of a piston rod and the plunger is minimized or even reduced to zero.

State of the art pen-type drug delivery devices that incorporate a dose setting feature often include a so-called end-of-content limiter to prevent a user from selecting a size of a dose which exceeds the amount of liquid drug remaining in a cartridge of the device. References WO 01/19434 A1, WO 2006/128794 A2 and WO 2010/149209 A1 include disclosure of such end-of-content limiters. For such devices manufacturing tolerances generally has the effect that the total doseable amount of liquid drug from a held cartridge varies across individual pen samples. This is an issue that potentially may appear confusing to the end-user.

Further related disclosures include references WO 09/024562 A1 and WO 2015/016547 A1.

### SUMMARY

It is an object of the present invention to provide a drug delivery device featuring improved and facilitated clearance reduction or even clearance elimination. It is a further object of the invention to provide a simplified and robust method of eliminating clearance in a drug delivery device. Finally, it is an object of the invention to provide manufacture of drug delivery devices providing consistently uniform and predictable total doseable amount of liquid drug from a held cartridge.

In the disclosure of the present invention, embodiments and aspects will be described which will address one or more of the above objects or which will address objects apparent from the below disclosure as well as from the description of exemplary embodiments.

In a first aspect, the present invention relates to a method of assembling a drug delivery device which in the assembled state comprises a piston equipped cartridge holding a drug and an expelling mechanism comprising a piston rod for providing a force on the piston. The method of assembling comprises the steps of:
a) providing a cartridge, the cartridge comprising a cartridge body having a distal outlet being closed by a penetrable septum and having a proximal open end closed by a slidable piston,
b) providing a piston rod for transferring a force to the piston,
c) arranging the cartridge and the piston rod along an axis,
d) providing a monitoring arrangement configured for detecting a change in geometry of the septum of the cartridge,
e) providing relative axial movement between the piston rod and the cartridge body for causing the piston rod to move the piston towards the septum causing the septum to deflect, and
f) interrupting said relative axial movement between the piston rod and the cartridge body when the septum assumes a predetermined geometry change.

As the piston rod moves the piston towards the septum an increasing load exerted by the piston rod is exerted on the cartridge. By accurately monitoring the deflection of the septum away from its initial configuration, a predefined preload of the piston of the cartridge is obtained. With the aim of keeping the preload during later storage, the preload is selected having a level where no or only little fluid will be expelled once an end-user attaches an injection needle to the device.

The cartridge may be of the kind wherein a distal outlet of the cartridge comprises a cylindrical section and wherein the septum, in a non-deflected state, defines a generally planar disc-shaped member comprising a central portion and a peripheral portion. The septum of the cartridge is configured so that the septum increasingly deflects upon increasing fluid pressure in the cartridge.

In some embodiments, a piston washer is arranged between the piston rod and the piston of the cartridge.

The method may be so arranged that in method step e), the piston moves towards the septum while the septum assumes a non-penetrated state.

In some embodiments the expelling mechanism of the assembled drug delivery device defines a manually operable expelling mechanism that does not comprise an electrically actuated drive mechanism.

In some embodiments the method of assembling further comprises the steps of:
d1) providing an electronically controlled actuating arrangement, the actuating arrangement being configured for causing said relative axial movement between the piston rod and the cartridge body, and
d2) providing a controller coupled to the actuating arrangement and the monitoring arrangement,
and wherein in method steps e) and f) the steps comprises operating the controller to operate the actuating arrangement to provide relative axial movement between the piston rod and the cartridge body and cease said movement when the septum assumes the predetermined geometry change as detected by the monitoring arrangement.

In some embodiments, prior to method step e), the method further comprises the step of enabling the expelling mechanism to operate the piston rod, and in method step e) operating the expelling mechanism to cause said relative axial movement between the piston rod and the cartridge body. Hence, by operating a subassembly comprising the expelling mechanism and the cartridge, the preloading adjustment may be made by operating the expelling mechanism.

In some embodiments, prior to method step e), the drug delivery device may be provided in the fully assembled state. In such embodiment, the fully assembled state may define a state where a protective cap has not yet been attached to cover the septum of the device. In such embodiments, the fully assembled drug delivery device may be manipulated subsequent to assembly operations to obtain a predefined geometry change for the septum.

In alternative embodiments, the drug delivery device defines cooperating first and second assembly adjustment components configured for being moved relative to each other during assembling, wherein, in step e) the first and second assembly adjustment components are moved relative to each other to cause said relative axial movement between the piston rod and the cartridge body, and wherein the method, subsequent to method step f), further comprises a step g) of permanently securing the first and second assembly adjustment components relative to each other to prevent the first and second assembly adjustment components from being operated relative to each other in a manner causing further relative axial movement between the piston rod and the cartridge body. In such embodiments, the relative axial movement between the piston rod and the cartridge body for obtaining a predefined geometry change for the septum is performed by components which are fixedly attached relative to each other during normal dose setting and dose expelling operations for the final assembled device.

Exemplary, non-limiting examples of first and second assembly adjustment components for causing said relative axial movement between the piston rod and the cartridge body may include first and second piston rod parts that are adjusted relative to each other during step e). Other exemplary first and second assembly adjustment components may comprise first and second parts adjusting the cartridge relative to the expelling mechanism. Still other exemplary first and second assembly adjustment components may comprise first and second parts adjusting the expelling mechanism fully, or a part thereof, relative to a housing of the device. Still other exemplary first and second assembly adjustment components may comprise first and second parts adjusting a distal housing part relative to a proximal housing part of the device.

In some embodiments the monitoring arrangement may comprise an imaging device recording the shape of the septum and wherein, in method steps e) and f), as the piston is moved towards the septum, operating the monitoring arrangement by optically detecting geometry changes of the septum.

The monitoring arrangement may comprise an illumination source for illuminating the septum area of the device. The illumination source may generate a pattern of illumination on a surface of the septum where the pattern of illumination comprising a line extending from the central portion towards a peripheral portion of the septum. The imaging device may be located offset from the axis and at a location where said line increasingly deviates from a linear shape as the septum deflection is increased. The imaging device may be oriented at an inclined angle to the septum at a location transverse to said line.

Other monitoring arrangement may comprise a linear position meter arranged relative to or coupled to the septum. The linear position meter may be in contact with the distal surface of the septum and be configured to follow and monitor axial movement of the septum as the piston is moved towards the septum.

In other embodiments the monitoring arrangement comprises a force transducer coupled to the septum, the force transducer being configured to detect an increased axial force exerted by the septum as the piston is moved towards the septum.

Subsequently to method step f), wherein said relative axial movement between the piston rod and the cartridge body is interrupted, the method comprises the further steps of inserting the fully assembled drug delivery device in a secondary packaging and sealing the secondary packaging. The method may include storing the device in the sealed secondary packaging while maintaining the relative axial position between the piston and the septum unaltered relative to the relative axial position as provided in method step f).

In exemplary embodiments for a device holding a cartridge, such as a 3 ml Penfill® cartridge or a 3ml "Standard A" cartridge containing a 100 IU/ml insulin formulation, a suitable aimed preload may correspond to an axial displacement of the piston washer in the order of 0.2-0.3 mm, measured from the initial condition where the piston rod, by means of the piston washer, starts to engage the piston.

In other exemplary embodiments, subsequent to step e), a preloading of the cartridge piston is provided corresponding to axial movement of the piston by a displacement corresponding to expelling 0.5 to 2 IU of liquid from the cartridge if a needle were immediately attached.

In still other exemplary embodiments, subsequent to step e), a preloading of the piston of the cartridge is provided corresponding to a displacement of the piston for expelling a quantity of liquid between 0.5 to 1 IU of liquid from the cartridge (if a needle were immediately attached).

In a second aspect, the present invention relates to a drug delivery device assembled in accordance with any of the methods described herein.

As used herein, the term "insulin" is meant to encompass any drug-containing flowable medicine capable of being passed through a delivery means such as a cannula or hollow needle in a controlled manner, such as a liquid, solution, gel or fine suspension, and which has a blood glucose controlling effect, e.g. human insulin and analogues thereof as well as non-insulins such as GLP-1 and analogues thereof. In the description of exemplary embodiments reference will be made to the use of insulin.

### BRIEF DESCRIPTION OF DRAWINGS

In the following the invention will be further described with reference to the drawings, wherein
figs. 1A and 1B are perspective views of a prior art pen device with and without a protective cap attached,
fig. 2 shows in an exploded view the components of the pen device of figs. 1A and 1B,
figs. 3A and 3B show in sectional views of an expelling mechanism of the pen device of figs. 1A and 1B in two different pen modes,
fig. 4 schematically represents a piston deflection monitoring arrangement according to the invention involving a linear displacement sensor,
figs. 5A, 5B and 5C schematically represent three embodiments of a septum deflection monitoring arrangement according to the invention involving optical sensor arrangements,
figs. 5D and 5E schematically represent two embodiments of a septum deflection monitoring arrangement according to the invention involving laser scanning arrangements,
figs. 6a and 6B show examples of septum bulging as a function of load on the piston of a held cartridge,
figs. 7a and 7B show examples of septum bulging as a function of piston washer movement relative to a held cartridge in fully assembled injection pens,
fig. 8 shows the septum deflection as a function of dialled and "delivered" single units, i.e. "clicks".
fig. 9 shows a height profile data for the septum area of a preloaded pen obtained with laser scanning arrangement similar to the arrangement shown in fig. 5C,
fig. 10 shows septum deflection for a series of sample pens with three successive 1-unit dose deliveries,
fig. 11 is a statistical representation of the data of fig. 10 providing normal distributions,
fig. 12 is a perspective view of main components for a first alternative pen device assembled in accordance with the invention, and
fig. 13A through 13F show different assembly states during assembly of the pen device shown in fig 12.

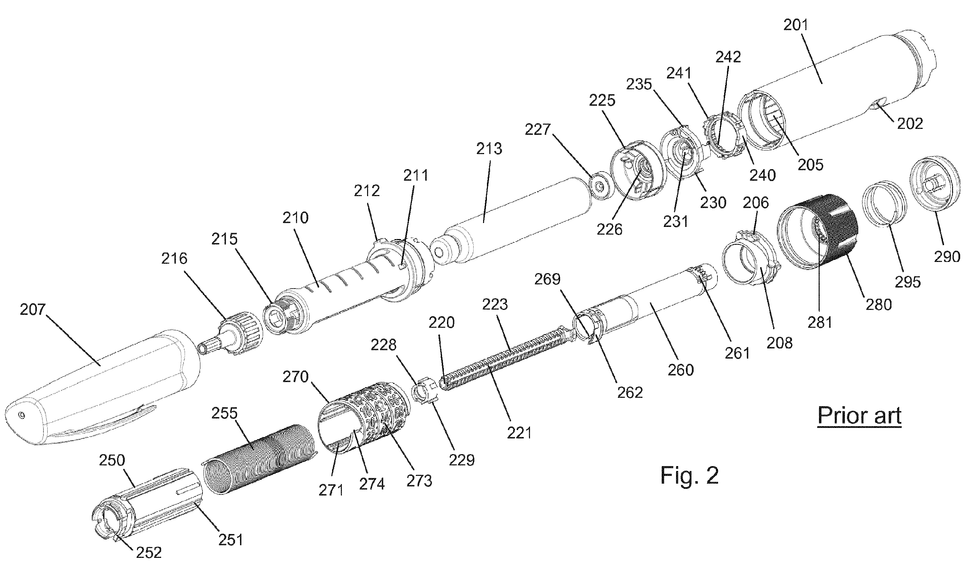

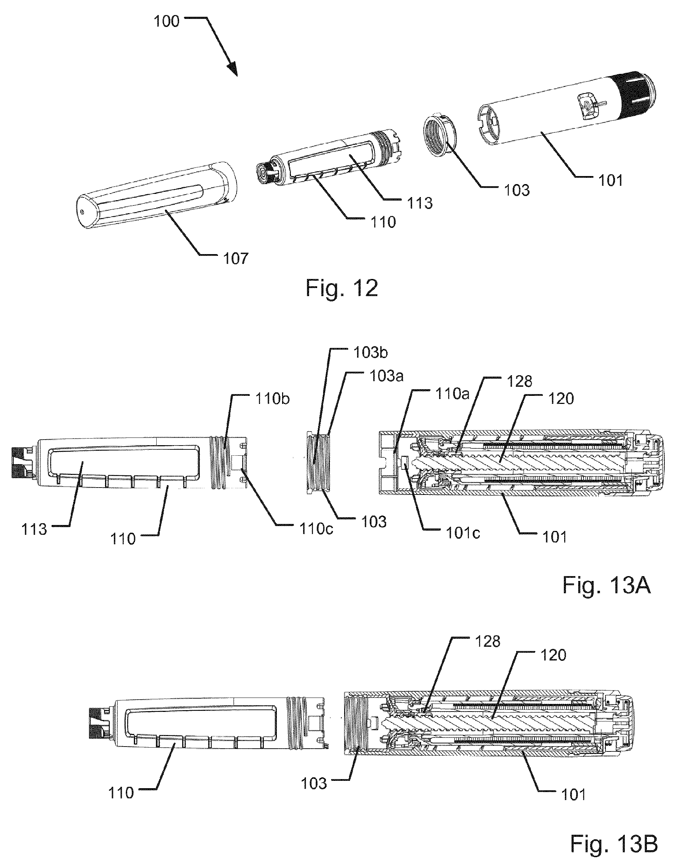

In the figures corresponding structures are mainly identified by corresponding reference numerals, i.e. whereby parts carrying reference no. "220" in fig. 2 corresponds to reference "120" in figs. 12 through 13F, etc.

### DESCRIPTION

When in the following terms such as "upper" and "lower", "right" and "left", "horizontal" and "vertical" or similar relative expressions are used, these only refer to the appended figures and not necessarily to an actual situation of use. The shown figures are schematic representations for which reason the configuration of the different structures as well as their relative dimensions are intended to serve illustrative purposes only. When the term member or element is used for a given component it generally indicates that in the described embodiment the component is a unitary component, however, the same member or element may alternatively comprise a number of sub-components just as two or more of the described components could be provided as unitary components, e.g. manufactured as a single injection moulded part. The term "assembly" does not imply that the described components necessarily can be assembled to provide a unitary or functional assembly during a given assembly procedure but is merely used to describe components grouped together as being functionally more closely related.

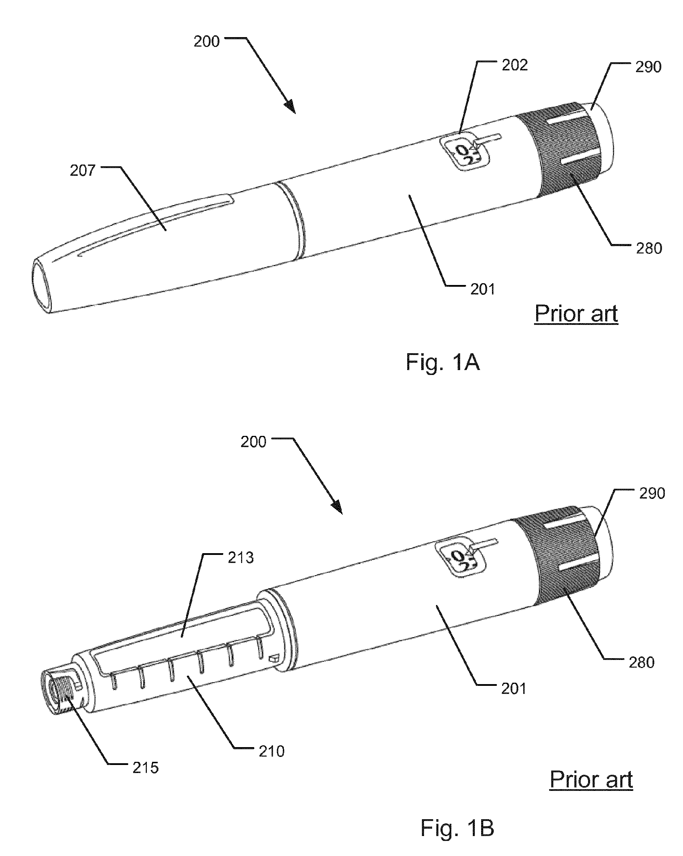

Figs. 1A and 1B show a prior art drug delivery device in the form of a pen-formed auto-injection device 200, i.e. a so-called "injection pen" that includes an expelling mechanism incorporating a spring drive. Fig. 2 shows an exploded view of the prior art auto-injection device 200 shown in figs. 1A and 1B.

In the present context the device 200 represents a "generic" drug delivery device providing a specific example of a device which may be assembled using the assembly method of the present invention. The example shown in figs. 1 through 3B shows a drug delivery device of the pre-filled type, i.e. it is supplied with a pre-mounted cartridge and is to be discarded when the cartridge has been emptied. In alternative embodiments, and in accordance with the present invention, the drug delivery device may be designed to allow a loaded cartridge to be inserted, e.g. in the form of a "rear-loaded" drug delivery device in which the cartridge holder is adapted to be attached to the device main portion, or alternatively in the form of a "front-loaded" device in which a cartridge is inserted through a distal opening in the cartridge holder which is non-removable attached to the main part of the device.

The pen device 200 comprises a cap part 207 and a main part relative to which the cap part is removably attachable. The main part includes a proximal body or drive assembly portion having a housing 201 accommodating a drug expelling mechanism. The main part further includes a distal cartridge holder portion 210 in which a drug-filled cartridge 213 is retained. The cartridge comprises a proximal cylindrical section accommodating a drug and a distal outlet having a needle-penetrable septum arranged for sealing the distal end of the cartridge. The proximal cylindrical section is sealed towards its proximal end by a piston which is axially slideably retained within the cartridge. The cartridge may for example contain an insulin, GLP-1 or growth hormone formulation. The cartridge holder includes openings allowing a portion of the cartridge to be inspected. The cartridge holder also includes distal coupling means 215 allowing a needle assembly to be releasably mounted in a way enabling fluid communication with the cartridge interior. The cartridge piston is configured for being driven by a piston rod being coupled to the expelling mechanism. A proximal-most rotatable dose dial member 280 serves to manually set a desired dose of drug shown in display window 202 and which can then be expelled when the release button 290 is actuated. As shown in this embodiment, the expelling mechanism may comprise a spring which is strained during dose setting and then released to drive the piston rod when the release button 290 is actuated. Alternatively the expelling mechanism may be fully manual in which case the dose dial member and the actuation button moves proximally during dose setting corresponding to the set dose size, and then is moved distally by the user to expel the set dose.

Fig. 2 shows an exploded view of the pen-formed drug delivery device 200 shown in fig. 1. More specifically, the pen comprises a tubular housing 201 with a window opening 202 and onto which a cartridge holder 210 is fixedly mounted, a drug-filled cartridge 213 being arranged in the cartridge holder. The cartridge holder is provided with distal coupling means 215 allowing a needle assembly 216 to be releasably mounted, proximal coupling means in the form of two opposed protrusions 211 allowing cap 207 to be releasably mounted covering the cartridge holder and a mounted needle assembly, as well as a protrusion 212 preventing the pen from rolling on e.g. a table top. In the distal end of housing 201 a nut element 225 is fixedly mounted, the nut element comprising a central threaded bore 226, and in the housing proximal end a spring base member 208 with a central opening is fixedly mounted. A drive system comprises a piston rod 220 that includes two opposed longitudinal grooves 221 and threads 223 provided along its longitudinal extension. The piston rod 220 is received in the nut element threaded bore 226. A ring-formed piston rod drive element 230 is rotationally arranged in the housing, and a ring-formed clutch element 240 is in rotational engagement with the drive element (see below), the engagement allowing axial movement of the clutch element. The clutch element is provided with outer spline elements 241 adapted to engage corresponding splines (not shown) on the housing inner surface, this allowing the clutch element to be moved between a rotationally locked proximal position, in which the splines are in engagement, and a rotationally free distal position in which the splines are out of engagement. As just mentioned, in both positions the clutch element 240 is rotationally locked to the drive element 230. The drive element comprises a central bore with two opposed protrusions 231 in engagement with the grooves 221 on the piston rod whereby rotation of the drive element results in rotation and thereby distal axial movement of the piston rod due to the threaded engagement between the piston rod and the nut element. The drive element further comprises a pair of opposed circumferentially extending flexible ratchet arms 235 adapted to engage corresponding ratchet teeth 205 arranged on the housing inner surface. The drive element and the clutch element comprise cooperating coupling structures rotationally locking them together but allowing the clutch element to be moved axially, this allowing the clutch element to be moved axially to its distal position in which it is allowed to rotate, thereby transmitting rotational movement from the dial system (see below) to the drive system.

On the piston rod an end-of-content (EOC) member 228 (EOC limiter) is threadedly mounted and on the distal end a washer 227 is rotationally mounted. The EOC member comprises a pair of opposed radial projections 229 for engagement with the reset tube (see below).

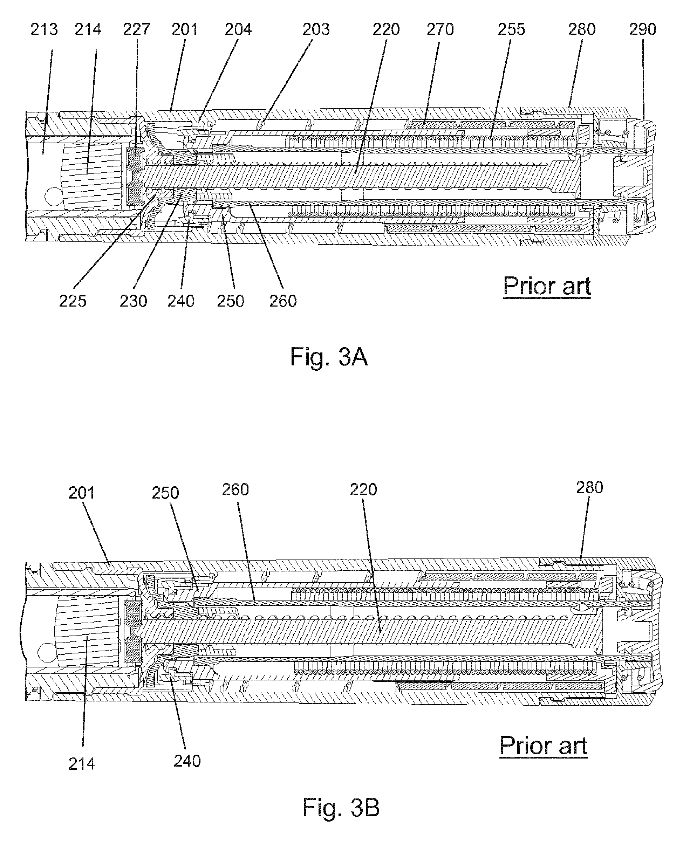

The dial system comprises a ratchet tube 250, a reset tube 260, a scale drum 270 with an outer helically arranged row of dose numerals, a user-operated dose dial member 280 for setting a dose of drug to be expelled, a release button 290 and a torque spring 255 (see fig. 3A and 3B). The reset tube is mounted axially locked inside the ratchet tube but is allowed to rotate a few degrees (see below). The reset tube comprises on its inner surface two opposed longitudinal grooves 269 adapted to engage the radial projections 229 of the EOC member, whereby the EOC can be rotated by the reset tube but is allowed to move axially. The clutch element is mounted axially locked on the outer distal end portion of the ratchet tube 250, this providing that the ratchet tube can be moved axially in and out of rotational engagement with the housing via the clutch element. The dose dial member 280 is mounted axially locked but rotationally free on the housing proximal end, the dose dial member being under normal operation rotationally locked to the reset tube (see below), whereby rotation of dose dial member results in a corresponding rotation of the reset tube and thereby the ratchet tube. The release button 290 is axially locked to the reset tube but is free to rotate. A return spring 295 provides a proximally directed force on the button and the thereto mounted reset tube. The scale drum 270 is arranged in the circumferential space between the ratchet tube and the housing, the drum being rotationally locked to the ratchet tube via cooperating longitudinal splines 251, 271 and being in rotational threaded engagement with the inner surface of the housing via cooperating thread structures 203, 273, whereby the row of numerals passes the window opening 202 in the housing when the drum is rotated relative to the housing by the ratchet tube. The torque spring is arranged in the circumferential space between the ratchet tube and the reset tube and is at its proximal end secured to the spring base member 208 and at its distal end to the ratchet tube, whereby the spring is strained when the ratchet tube is rotated relative to the housing by rotation of the dial member. A ratchet mechanism with a flexible ratchet arm 252 is provided between the ratchet tube and the clutch element, the latter being provided with an inner circumferential teeth structures 242, each tooth providing a ratchet stop such that the ratchet tube is held in the position to which it is rotated by a user via the reset tube when a dose is set. In order to allow a set dose to be reduced a ratchet release mechanism 262 is provided on the reset tube and acting on the ratchet tube, this allowing a set dose to be reduced by one or more ratchet increments by turning the dial member in the opposite direction, the release mechanism being actuated when the reset tube is rotated the above-described few degrees relative to the ratchet tube.

Having described the different components of the expelling mechanism and their functional relationship, operation of the mechanism will be described next with reference mainly to figs. 3A and 3B where fig. 3A shows the device in a dose setting mode and fig. 3B shows the device in a dose expelling mode.

The pen mechanism can be considered as two interacting systems, a dose system and a dial system, this as described above. In dose setting mode, during dose setting the dial mechanism rotates and a torsion spring of the spring drive is loaded. The dose mechanism is locked to the housing and cannot move. When the push button is pushed down, the pen will change into dose expelling mode where the dose mechanism is released from the housing and due to the engagement to the dial system, the torsion spring will now rotate back the dial system to the starting point and rotate the dose system along with it.

The central part of the dose mechanism is the piston rod 220, the actual displacement of the piston being performed by the piston rod. During dose delivery, the piston rod is rotated by the drive element 230 and due to the threaded interaction with the nut element 225 which is fixed to the housing, the piston rod moves forward in the distal direction. Between the rubber piston 214 and the piston rod 220, the piston washer 227 is placed which serves as an axial bearing for the rotating piston rod and evens out the pressure on the rubber piston. As the piston rod has a non-circular cross section where the piston rod drive element engages with the piston rod, the drive element is locked rotationally to the piston rod, but free to move along the piston rod axis. Consequently, rotation of the drive element results in a linear forward movement of the piston. The drive element is provided with small ratchet arms 234 which prevent the drive element from rotating clockwise (seen from the push button end). Due to the engagement with the drive element, the piston rod can thus only move forwards. During dose delivery, the drive element rotates anticlockwise and the ratchet arms 235 provide the user with small clicks due to the engagement with the ratchet teeth 205, e.g. one click per unit of insulin expelled.

Turning to the dial system, the dose is set and reset by turning the dose dial member 280. When turning the dial, the reset tube 260, the EOC member 228, the ratchet tube 250 and the scale drum 270 all turn with it. As the ratchet tube is connected to the distal end of the torque spring 255, the spring is loaded. During dose setting, the arm 252 of the ratchet performs a dial click for each unit dialled due to the interaction with the inner teeth structure 242 of the clutch element. In the shown embodiment the clutch element is provided with 24 ratchet stops providing 24 clicks (increments) for a full 360 degrees rotation relative to the housing. The spring is preloaded during assembly which enables the mechanism to deliver both small and large doses within an acceptable speed interval. As the scale drum is rotationally engaged with the ratchet tube, but movable in the axial direction and the scale drum is in threaded engagement with the housing, the scale drum will move in a helical pattern when the dial system is turned, the number corresponding to the set dose being shown in the housing window 202.

The ratchet 252, 242 between the ratchet tube and the clutch element 240 prevents the spring from turning back the parts. During resetting, the reset tube moves the ratchet arm 252, thereby releasing the ratchet click by click, one click corresponding to one unit IU of insulin in the described embodiment. More specifically, when the dial member is turned clockwise, the reset tube simply rotates the ratchet tube allowing the arm of the ratchet to freely interact with the teeth structures 242 in the clutch element. When the dial member is turned counter-clockwise, the reset tube interacts directly with the ratchet click arm forcing the click arm towards the centre of the pen away from the teeth in the clutch, thus allowing the click arm on the ratchet to move "one click" backwards due to torque caused by the loaded spring.

To deliver a set dose, the release button 290 is pushed in the distal direction by the user as shown in fig. 3B. The reset tube 260 decouples from the dial member and subsequently the clutch element 240 disengages the housing splines. Now the dial mechanism returns to "zero" together with the drive element 230, this leading to a dose of drug being expelled. It is possible to stop and start a dose at any time by releasing or pushing the push button at any time during drug delivery. A dose of less than 5 IU normally cannot be paused, since the rubber piston is compressed very quickly leading to a compression of the rubber piston and subsequently delivery of insulin when the piston returns to the original dimensions.

The EOC feature prevents the user from setting a larger dose than left in the cartridge. The EOC member 228 is rotationally locked to the reset tube, which makes the EOC member rotate during dose setting, resetting and dose delivery, during which it can be moved axially back and forth following the thread of the piston rod. When it reaches the proximal end of the piston rod a stop is provided, this preventing all the connected parts, including the dial member, from being rotated further in the dose setting direction, i.e. the now set dose corresponds to the remaining drug content in the cartridge.

The scale drum 270 is provided with a distal stop surface 274 adapted to engage a corresponding stop surface on the housing inner surface, this providing a maximum dose stop for the scale drum preventing all the connected parts, including the dial member, from being rotated further in the dose setting direction. In the shown embodiment the maximum dose is set to 80 IU. Correspondingly, the scale drum is provided with a proximal stop surface adapted to engage a corresponding stop surface on the spring base member, this preventing all the connected parts, including the dial member, from being rotated further in the dose expelling direction, thereby providing a "zero" stop for the entire expelling mechanism. In the following, the position that the dial member assumes after completion of the expelling of a set dose will be referred to as the "zero-position".

To prevent accidental over-dosage in case something should fail in the dialing mechanism allowing the scale drum to move beyond its zero-position, the EOC member serves to provide a security system. More specifically, in an initial state with a full cartridge the EOC member is positioned in a distal-most axial position in contact with the drive element. After a given dose has been expelled the EOC member will again be positioned in contact with the drive element. Correspondingly, the EOC member will lock against the drive element in case the mechanism tries to deliver a dose beyond the zero-position. Due to tolerances and flexibility of the different parts of the mechanism the EOC will travel a short distance allowing a small "over dose" of drug to be expelled, e.g. 3-5 IU of insulin.

The expelling mechanism further comprises an end-of-dose (EOD) click feature providing a distinct feedback at the end of an expelled dose informing the user that the full amount of drug has been expelled. More specifically, the EOD function is made by the interaction between the spring base and the scale drum. When the scale drum returns to zero, a small click arm 206 on the spring base is forced backwards by the progressing scale drum. Just before "zero" the arm is released and the arm hits a countersunk surface on the scale drum.

The shown mechanism is further provided with a torque limiter in order to protect the mechanism from overload applied by the user via the dose dial member. This feature is provided by the interface between the dose dial member and the reset tube which as described above are rotationally locked to each other. More specifically, the dose dial member is provided with a circumferential inner teeth structure 281 engaging a number of corresponding teeth arranged on a flexible carrier portion 261 of the reset tube. The reset tube teeth are designed to transmit a torque of a given specified maximum size, e.g. 150-300 Nmm, above which the flexible carrier portion and the teeth will bend inwards and make the dose dial member turn without rotating the rest of the dial mechanism. Thus, the mechanism inside the pen cannot be stressed at a higher load than the torque limiter transmits through the teeth.

Production tolerances on the piston rod and the remaining parts of the expelling mechanism, the cartridge body, cartridge filling level and other components result in variations in piston rod position and piston position in each device during assembly. Hence, with no adjustments the final assembled pen samples would exhibit varying levels of clearance between the piston and the piston rod.

In mechanical devices production, in order to minimize a potential clearance between the piston rod and the piston of the cartridge, positioning may be carried out by initially positioning the piston rod in a nominal position. In the example pen device described above, during assembly, the pen may be arranged as a distal subassembly holding the cartridge and the washer and a proximal subassembly holding the expelling mechanism including the piston rod. Due to tolerances various different clearance gaps between the piston/washer and the piston rod will show when the distal and proximal subassemblies of each pen sample are permanently secured together. On the basis of measurements or estimations, which may be performed at different steps of the assembly process, the actual clearance or gap in each sample may be eliminated or at least partly reduced by operating the expelling mechanism. Operating the expelling mechanism may be carried out either after final assembly or prior to final assembly of the different subassemblies. However such compensation procedure means that the end-of-content mechanism will be operated to a lesser or higher degree even before the device is shipped to the user meaning that the experienced total doseable volume varies from sample to sample. Generally such variations and inconsistencies from one sample to another should be avoided as this may provide the impression that the quality of the device could be somewhat flawed.

In accordance with the present invention, a method of consistently eliminating or reducing clearance will be described. The method relies on the principle of, during manufacturing and final assembly, providing relative axial movement between the piston rod and the cartridge body for causing the piston rod to move the piston towards the septum, and to retain this state for long term storage. With the septum of the cartridge assuming the sealed, i.e. the non-penetrated state, after an initial compression of the piston within the cartridge, the piston will eventually move axially inside the cartridge and cause the septum to deflect as fluid pressure in the cartridge increases.

In certain embodiments, the septum is mounted onto the neck of the cartridge body by means of a septum cap. As the cartridge is provided for assembly into the drug delivery device, the cartridge septum will assume an initial configuration with the piston in a steady non-loaded condition. In this state the septum may assume a non-deflected, or in a substantially non-deflected configuration mainly being influenced by the septum cap.

During increasing load exerted by the piston rod, by accurately monitoring the deflection of the septum away from its initial configuration, a controlled pressure build up in the cartridge can be ensured with the aim of keeping the preload during later storage, the preload having a level where no or only little fluid will be expelled once an end-user attaches an injection needle to the device.

In exemplary embodiments for a 3 mL Penfill® cartridge containing a 100 IU/ml insulin formulation, a suitable aimed pressure may correspond to an axial displacement of the piston washer in the order of 0.2-0.3 mm, measured from the initial condition where the piston rod, by means of the piston washer, starts to engage the piston.

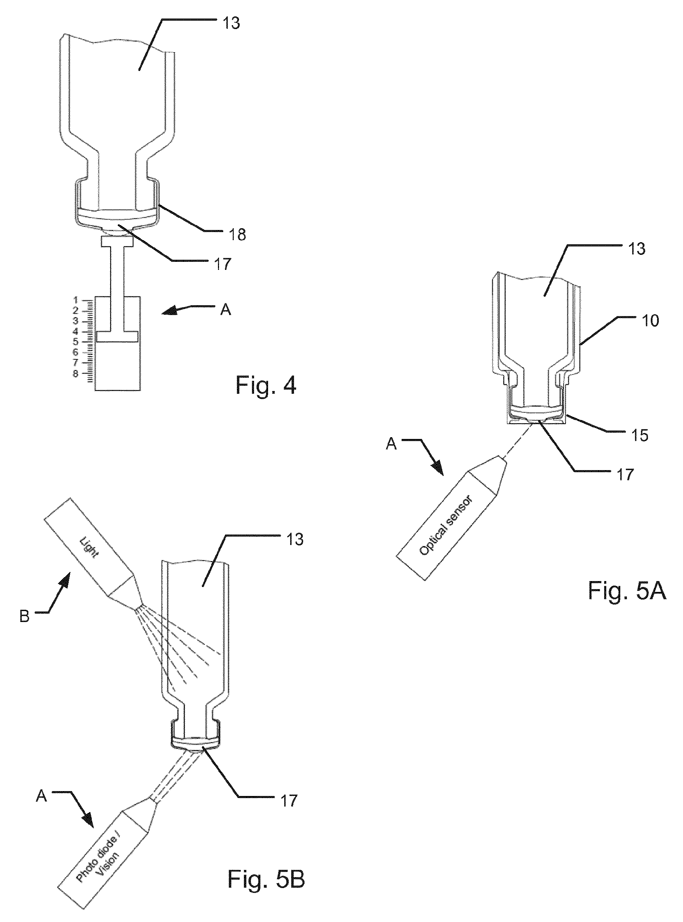

In one embodiment, a displacement sensor is used for monitoring axial deflection of the central part of the septum away from its initial configuration. As schematically shown in fig. 4 such displacement sensor may be provided as a contact displacement sensor in the form of a linear position meter "A" arranged along the axis of the cartridge 13 and in contact with the piston septum 17 at the distal central surface thereof. In other embodiments other types of displacement sensors such as optical or ultrasonic sensors may be used. In fig. 4 the septum cap 18 can be viewed.

In other embodiments using optically based sensors, the optical displacement sensor "A" may be arranged to sense the distal surface of the septum 17, this being schematically shown in fig. 5A. A suitable illumination source will typically be arranged in such setup for properly illuminating the target area, for example illumination the area of interest from the distal side. Alternatively, when the septum 17 is at least partially translucent, illumination may be provided from the rear of the septum where an illumination source "B" may be arranged to radiate through windows or openings of the cartridge holder 10 and radiate through the transparent walls of the medicament cartridge, see fig. 5B.

A bulging septum may be best viewed from directions transverse to the cartridge axis. However, as shown in fig. 5A, for some embodiments the assembled injection pen may include a needle interface 15 for connecting an injection needle to the device. The geometry of the needle interface 15 may obstruct the bulging septum from being observed at right angles to the cartridge axis. Therefore optical sensors may be arranged to focus on the cartridge septum at angles inclined with respect to the central axis of the cartridge.

Other exemplary embodiments may use vision technology for monitoring bulging of the septum of the cartridge as the internal liquid pressure builds up. The monitoring arrangement may comprise an illumination source generating a pattern of illumination on a surface of the septum and may further comprise an image sensor that records images of the illuminated septum. When the septum bulges, the light pattern on the septum surface will appear distorted when seen from an angle. Thus, by arranging the image sensor at an inclined angle relative to the axis of the cartridge, the vision arrangement may be configured to correlate the distorted pattern with the deflection of the septum and thus to correlate with the internally fluid pressure in the cartridge. In alternative embodiments, the septum may be provided with a patterned image printed thereon where the pattern will appear distorted with increasing bulging of the septum.

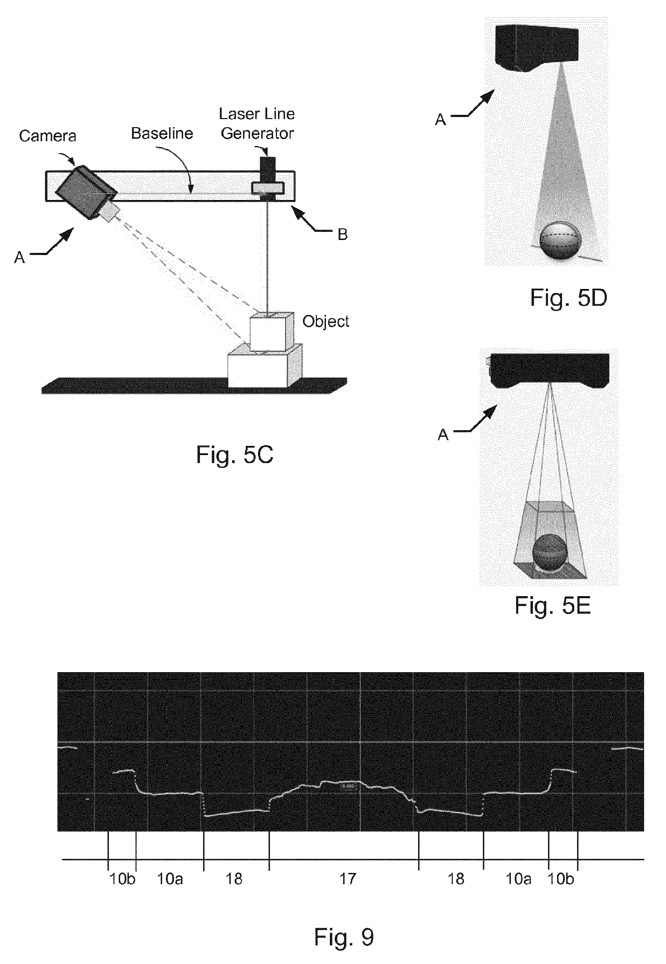

Fig. 5C shows a schematic representation of a further embodiment with a light source in the form of a laser line generator which is arranged to radiate towards the object and impinge a surface area of the object, e.g. the cartridge septum and neighbouring areas surrounding the septum, such as the cartridge holder, needle interface and/or fixture for the assembling machinery. The laser draws a line across the object and a camera obtains an image of this line seen from an angle. The result is a single line profile containing height information. In alternative embodiments providing fast data acquisition and fairly precise height measurement a corresponding setup may use a laser scanner apparatus such as depicted in fig. 5D.

In a further embodiment schematically shown in fig. 5E a different technique is used, namely structured light in combination with stereo vision. A single centred light source emits structured light onto the object which is captured by two cameras generating a 3D height map. The advantage of this technique is that height data from the entire object instead of just a single line is obtained.

In exemplary embodiments for a 3 ml Penfill® cartridge containing a 100 IU/ml insulin formulation, a suitable aimed preload may correspond to an axial displacement of the piston washer in the order of 0.2-0.3 mm, measured from the initial condition where the piston rod, by means of the piston washer, starts to engage the piston.

In one embodiment, by selecting a slight preloading corresponding to axial movement of the piston by a displacement corresponding to expelling 0.5 to 2 IU of liquid from the cartridge if a needle were immediately attached, only a slight expulsion of liquid will occur when an injection needle is later attached penetrating the septum. Subsequent to storage for a few weeks of the assembled pen in the pre-stressed condition with the preloaded cartridge, the expulsion of liquid when a needle is attached will be even reduced compared to a potential needle penetration of the septum right after assembly in the pre-stressed condition.

For certain embodiments, further preloading may be less favourable due to expulsion of too large an amount of liquid when the needle penetrates the septum for the first time. For some embodiments, a pre-stressing of the cartridge, corresponding to a displacement of the piston for expelling a quantity of liquid in the order of 0.5 to 1 IU of liquid from the cartridge (if a needle were immediately attached) will be suitable.

### Example 1

Measurements of different sample cartridges have been made for investigating the effect of preloading the cartridge and the septum deflection caused by the applied load.

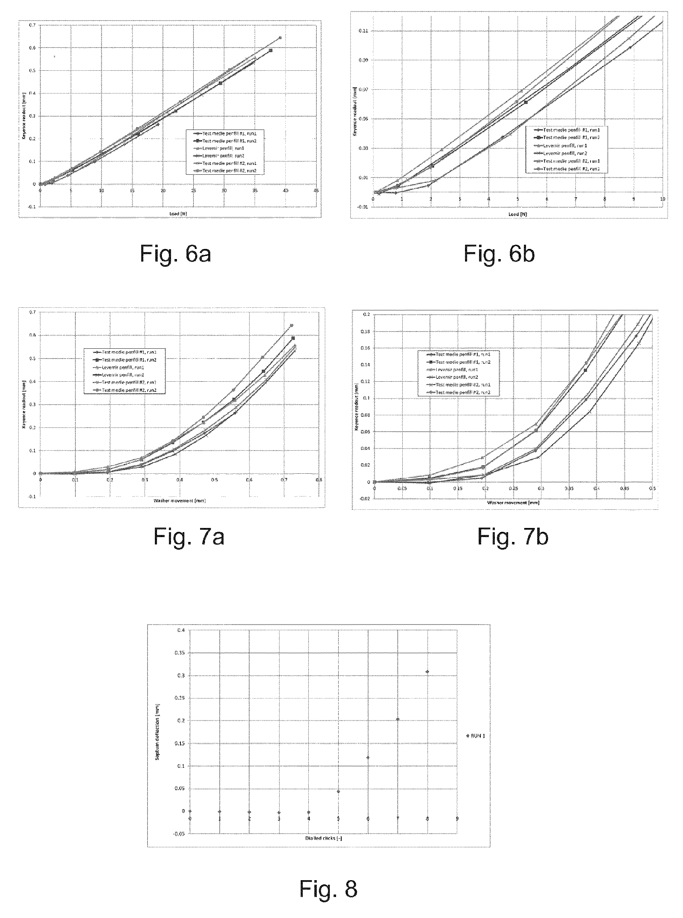

A series of PenFill 3 mL cartridges were installed, one at a turn, in a custom made fixture wherein load was increasingly exerted onto the piston and septum deflection was recorded using a linear displacement sensor corresponding to the monitoring arrangement schematically shown in fig. 4. An electrically controlled actuator has been used for providing a plunging force on the piston of the cartridge. Data are reported as the change in septum deflection as a function of load on the plunger. Fig. 6a shows the septum bulging as a function of the load on the piston for six different runs whereas fig. 6b shows a magnified view of the lower left corner of fig. 6a. Fig. 7a show corresponding views of the septum bulging as a function of the washer displacement whereas fig. 7b shows a magnified view of the lower left corner of fig. 7a. The curves shown in figs. 6a and 6b behave linearly and the bulging starts at a very low load. However, although the bulging is a linear function of the load, the non-linear response for the washer displacement is a result of the piston being significantly compressed before any noticeable septum bulging is observed.

### Example 2

Measurement of an assembled device similar to the injection pen described in figs. 1 to 3B accommodating a prefilled cartridge has been made, again using a linear displacement sensor corresponding to the monitoring arrangement schematically shown in fig. 4. The injection pen was arranged in a fixture and with no needle attached to the device so no expelling of liquid from the cartridge was possible. The device was subjected to a sequence of dial operations of a single unit with an attempted out-dosing operation after each dial operation. The septum deflection has been recorded continuously. Fig. 8 shows the septum deflection as a function of dialled and "delivered" single units, i.e. "clicks".

As no initial priming of the device was done prior to testing, the first four 1-unit dose deliveries did not influence the septum as read by the linear displacement sensor. When the fifth 1-unit dose was delivered the cartridge septum started bulging and for each subsequent click, the septum bulged further, with progressively larger changes in the bulge for each delivery operation. At the eight 1-unit dose delivery, the bulge was about 0.3 mm. The ninth 1-unit dose delivery failed because the load in the cartridge was too large for the torsion spring to overcome. Here, no initial priming was done and, as a result, septum bulging was delayed until 1-unit dose delivery #5. The initial four 1-unit dose deliveries illustrate that consistent contact between the piston rod, the washer and the piston has not initially been made. Although the deflection is not fully linear as a function of dosed clicks in the graph in fig. 8, the change is roughly estimated to 0.09mm/click from dialled click 5 to dialled click 7.

### Example 3

Fig. 9 shows a recorded image obtained with a monitoring arrangement corresponding to the laser scanner shown in fig. 5C. The recorded image shows the front section of the assembled pen where the cartridge piston is provided with a specific load exerted by the piston rod. The shown height profile contains a central dome shaped profile section 17 representing the free surface of the septum. The section 17 is surrounded by the septum cap 18 which again is surrounded by a first annular flat region 10a of the cartridge holder 10 and a ridge section 10b of the cartridge holder. At the radially outermost border locations a fixation tool is visible.

The fluid pressure internally in the cartridge will be correlated with the height profile of the profile section 17. Absolute height measurements are likely highly susceptible to movements of the pen itself. Therefore a reference measurement is added and the deflection of the septum is then measured relative to the reference. In one embodiment, the flat annular region 10a may be used as a reference.

With such a monitoring arrangement, the test sequence of Example 3 has been repeated for 18 different pen samples where contact between the piston rod and the piston/washer has initially been made. In this example an initial zero dose delivery operation and three successive 1-unit dose deliveries were made. Each pen has been measured 5 times giving a total of 90 measurements. Each measurement contains 4 sub measurements: neutral, 1 click, 2 clicks and 3 clicks. The neutral measurement is used as a reference for the following "click" measurements, which are calculated as the difference relative to that reading.

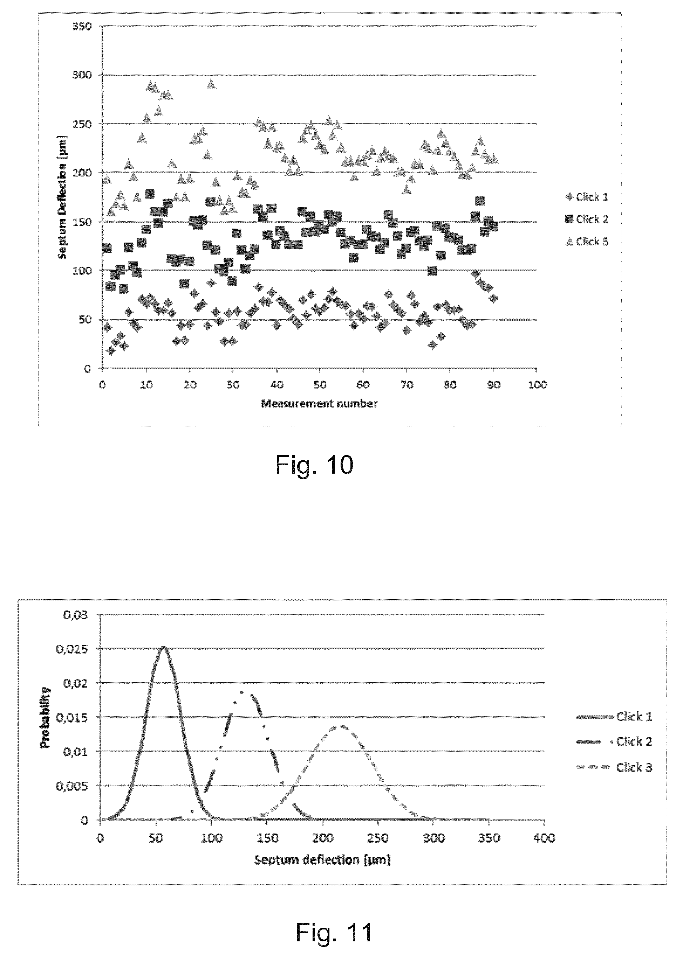

The measurements are shown in fig. 10. As shown in fig. 11, the distributions of the measurement series can be interpreted as normal distributions.

### END OF EXAMPLE

The above discussion of Example 1, Example 2 and Example 3 provide support for the relationship between piston rod axial force on the piston and the amount of bulging of the septum of the held cartridge. In order to take into consideration of a possible amount of air in the cartridges, the assembly method may be further adapted to continuously monitor piston movement and/or piston compression as the piston rod and the cartridge body is moved axially relative to each other. One non-limiting example of a means to monitor the piston location and/or piston compression is to include vision technology. For example if vision technology is used to capture both the change in the piston position, as well as the bulging of the septum, it enables a clearance elimination and preload adjustment that is insensitive to air in the cartridge. If an air bubble is present, significant piston movement can be expected with limited bulging of the septum, because an air bubble makes the cartridge system softer. If only little air is present, the system is relatively stiff and very little piston movement results in a relatively large pressure and, consequently, in a relatively large septum bulging.

Turning now to fig. 12, the main components of a first alternative exemplary embodiment of an auto-injection device 100 is shown. The auto-injection device 100 defines a proximal housing component 101 which accommodates an expelling mechanism comprising a spring-drive. The operating principle of the expelling mechanism may be designed in accordance with the prior art expelling mechanism described in connection with figs. 1A through 3B. However, in other embodiments, alternative spring-driven expelling mechanisms having other operating principles may be employed. Also manual injection devices configured for manually pressing forward a piston rod during dose expelling may be employed.

A distal housing component 110 holds a cartridge 113 comprising a liquid drug and with an axially slideable piston arranged therein. A cap part 107 is shown. Further a threaded adjustment component 103 is shown.

In the shown embodiment the threaded adjustment component 103 is configured to be axially held at an axial fixed position relative to the proximal housing component 101. Threaded adjustment component 103 is provided with a circumferential bead 103a to be received in a circumferential groove 101a formed in proximal housing component 101.

The threaded adjustment component 103 defines an inner thread 103b. The distal housing component 110 defines an outer thread 110b configured to engage the inner thread 103b.

I in proximal housing component 101 a plurality of radially inwards protruding axially extending ribs 101c are formed. Ribs 101c are configured for slideably being received in respective longitudinally extending slits 110c formed in distal housing component 110 at the proximal end face. Hence, distal housing component 110 cooperates with the proximal housing component 101 to enable relative axial displacement but prevent relative rotation.

In fig. 13A the above components including subcomponents are shown axially aligned. In fig. 13B, as a first assembly step, the threaded adjustment component 103 is inserted into groove 101a of proximal housing component 101.

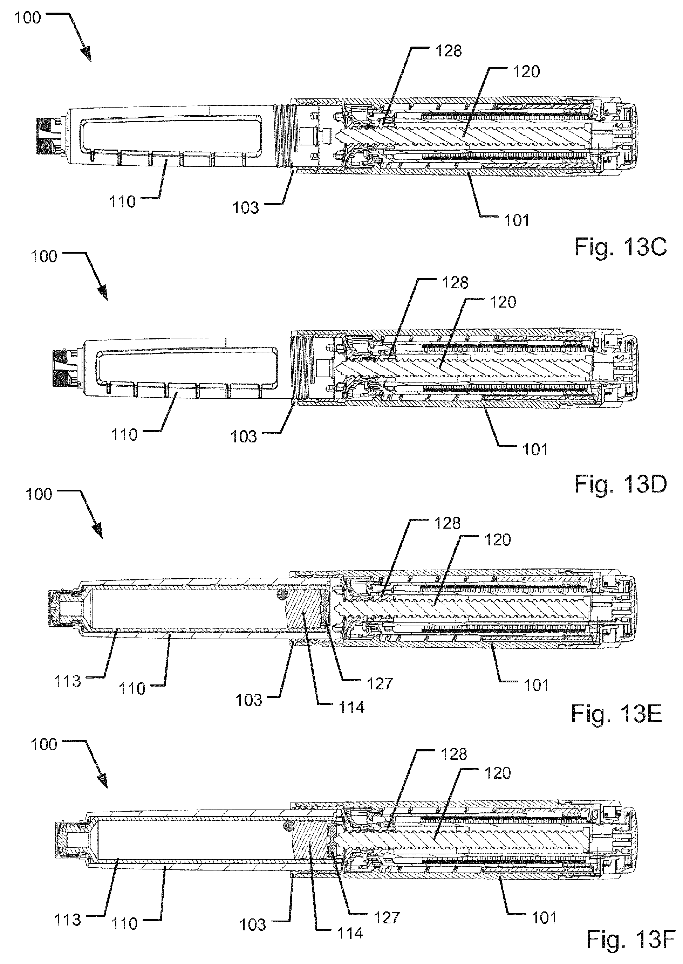

In fig. 13C the distal housing component 110 has been moved into sliding engagement with the proximal housing component 101. The threads 110b and 103b have not yet been engaged.

Fig 13D shows the distal housing component 110 has been moved further proximally in sliding engagement with the proximal housing component 101. The threads 110b and 103b have not yet been engaged.

In fig. 13E the threads 110b and 103b have only just been engaged. The distal end of the piston rod 120 is situated a distance from the piston washer situated next to the piston of the cartridge 113. By using a not shown tool, the threaded adjustment component 103 may be rotated to cause the distal housing component 110 to be moved further proximally. This has been carried out in fig. 13F where the piston washer has been caused to enter into abutment with the distal end of the piston rod 120. The movement may be performed under control of any type of measurement providing positioning feedback. Suitable methods may include optical measurements, force measurements or similar methods. If a clearance of predetermined magnitude is aimed at, optical measurements may be used or alternatively a force measurement may be used for ensuring abutment with subsequent predetermined axial movement creating the desired clearance. In accordance with the above discussion, the bulging of the septum of the cartridge may be continuously monitored for monitoring the pressure that the piston rod exerts on the piston. When the desired preload on the piston of the cartridge has been obtained the rotation of the threaded adjustment component 103 is interrupted

In some embodiments, the threaded engagement 110b/103b can only be rotated by means of a special tool and cannot be rotated by a human hand without the use of tools. Hence, a proper fastening method can be obtained. Alternatively, or in combination, other fastening methods can be used. For example the movement of the threaded adjustment component 103 may be immobilized by other means such as laser radiation, ultrasonic welding or similar fastening process either to the distal housing component 110, the proximal housing component 101 or both.

The assembly method has been provided so that the distal housing component 110 and the proximal housing component 101 has a proper rotational orientation relative to each other so that the intended rotational alignment between a window opening for the scale drum with the inspection openings of the cartridge holder is ensured. The assembled injection device 100 is now ready for labelling and other subsequent finalization steps. It is to be noted that during the assembly method above, the expelling mechanism has not been used. Hence, the EOC member 228 has been maintained at the same state throughout the assembly process.

In some embodiments, the above assembly method may include a "standard dose setting and expelling procedure" to be performed either before the states shown in fig. 13A or subsequently. The "standard dose setting and expelling procedure" may be the same involving the same amount of movement across the individual devices of the production line. Such dose setting and expelling procedure may for example be carried out for performing a function check for the expelling mechanism and/or for elimination of a previously established fixed "standard gap".

By using the described design and the assembly method, the clearance of each individual pen device can be effectively eliminated or alternatively reduced to a level which is uniform across individual pen devices. In situations where an end-of-content mechanism is incorporated into the device, the pen manufacturing and assembly can be carried out without use of the dose setting and expelling mechanism for reducing eliminating the gap between the piston rod and the piston and thus further enables the end-of-content mechanism of each individual device to provide a consistent and uniform total doseable quantity from each individual pen device.

The injection devices shown in figs. 1A through 3B and figs. 12 through 13F provides non-limiting examples of pen injectors which may be assembled according to the assembly method described herein. While certain features of the invention have been illustrated and described herein, many modifications, substitutions, changes, and equivalents will now occur to those of ordinary skill in the art. It is, therefore, to be understood that the appended claims are intended to cover all such modifications and changes as fall within the true spirit of the invention.

## Claims

1. A method of assembling a drug delivery device, the drug delivery device comprising a piston equipped cartridge holding a drug and an expelling mechanism comprising a piston rod for providing a force on the piston, the method of assembling comprising the steps of:
a) providing a cartridge, the cartridge comprising a cartridge body having a distal outlet being closed by a penetrable septum and having a proximal open end closed by a slidable piston,
b) providing a piston rod for transferring a force to the piston,
c) arranging the cartridge and the piston rod along an axis,
d) providing a monitoring arrangement configured for detecting a change in geometry of the septum of the cartridge,
e) providing relative axial movement between the piston rod and the cartridge body for causing the piston rod to move the piston towards the septum causing the septum to deflect, and
f) interrupting said relative axial movement between the piston rod and the cartridge body when the septum assumes a predetermined geometry change.

2. The method according to claim 1, wherein the distal outlet of the cartridge comprises a cylindrical section and wherein the septum, in a non-deflected state, defines a generally planar disc-shaped member comprising a central portion and a peripheral portion.

3. The method according to any of the claims 1-2, wherein, in method step e), the piston moves towards the septum while the septum assumes a non-penetrated state.

4. The method according to any of the claims 1-3, wherein the expelling mechanism of the assembled drug delivery device defines a manually operable expelling mechanism that does not comprise an electrically actuated drive mechanism.

5. The method according to any of the claims 1-4, wherein the method of assembling further comprises the steps of:
d1) providing an electronically controlled actuating arrangement, the actuating arrangement being configured for causing said relative axial movement between the piston rod and the cartridge body, and
d2) providing a controller coupled to the actuating arrangement and the monitoring arrangement,
and wherein in method steps e) and f) the steps comprises operating the controller to operate the actuating arrangement to provide relative axial movement between the piston rod and the cartridge body and cease said movement when the septum assumes the predetermined geometry change as detected by the monitoring arrangement.

6. The method according to any of the claims 1-5, wherein, prior to method step e), the method further comprises the step of enabling the expelling mechanism to operate the piston rod, and in method step e) operating the expelling mechanism to cause said relative axial movement between the piston rod and the cartridge body.

7. The method according to any of the claims 1-6, wherein prior to step e), the drug delivery device is provided in a fully assembled state.

8. The method according to any of the claims 1-7, wherein the drug delivery device defines cooperating first and second assembly adjustment components configured for being moved relative to each other during assembling, wherein, in step e) the first and second assembly adjustment components are moved relative to each other to cause said relative axial movement between the piston rod and the cartridge body, and wherein the method , subsequent to method step f), further comprises a step g) of permanently securing the first and second assembly adjustment components relative to each other to prevent the first and second assembly adjustment components from being operated relative to each other in a manner causing further relative axial movement between the piston rod and the cartridge body.

9. The method according to any of the claims 1-8, wherein the monitoring arrangement comprises an imaging device recording the shape of the septum and wherein, in method steps e) and f), as the piston is moved towards the septum, operating the monitoring arrangement by optically detecting geometry changes of the septum.

10. The method according to claim 9, wherein the monitoring arrangement comprises an illumination source generating a pattern of illumination on a surface of the septum, the pattern of illumination comprising a line extending from the central portion towards a peripheral portion of the septum, and wherein the imaging device is located offset from the axis and at a location where said line increasingly deviates from a linear shape as the septum deflection is increased.

11. The method according to any of the claims 1-10, wherein the monitoring arrangement comprises a linear position meter coupled to the septum and configured to follow and monitor axial movement of the septum as the piston is moved towards the septum.

12. The method according to any of the claims 1-11, wherein the monitoring arrangement comprises a force transducer coupled to the septum, the force transducer being configured to detect an increased axial force exerted by the septum as the piston is moved towards the septum.

13. The method according to any of the claims 1-12, wherein subsequently to method step f), wherein said relative axial movement between the piston rod and the cartridge body is interrupted, the method comprises the further steps of inserting the fully assembled drug delivery device in a secondary packaging and sealing the secondary packaging.

14. A drug delivery device assembled in accordance with the method as defined in any of the claims 1-13.
